(19) Europäisches Patentamt
European Patent Office
Office européen des brevets

(11) **EP 0 797 317 B1**

(12) **FASCICULE DE BREVET EUROPEEN**

(45) Date de publication et mention
de la délivrance du brevet:
**19.06.2002 Bulletin 2002/25**

(51) Int Cl.⁷: **H04B 5/00**, A61N 1/372

(21) Numéro de dépôt: **97400619.9**

(22) Date de dépôt: **20.03.1997**

(54) **Dispositif de réception de signaux émis par un appareil médical actif implanté**

Einrichtung zum Empfangen der Signale einer aktiven eingepflanzten medizinischen Anordnung

Receiving device for signals sent by an active implanted medical device

(84) Etats contractants désignés:
**BE CH DE FR GB IT LI SE**

(30) Priorité: **22.03.1996 FR 9603607**

(43) Date de publication de la demande:
**24.09.1997 Bulletin 1997/39**

(73) Titulaire: **ELA MEDICAL (Société anonyme)
F-92541 Montrouge (FR)**

(72) Inventeurs:
• **Lee, Chik Yam
94110 Arcueil (FR)**
• **Deschamp, Hervé
92150 Suresnes (FR)**

(74) Mandataire: **Dupuis-Latour, Dominique et al
Avocat à la Cour,
Cabinet Bardehle, Pagenberg & Partner,
14 boulevard Malesherbes
75008 Paris (FR)**

(56) Documents cités:
EP-A- 0 661 077        DE-A- 3 936 547
US-A- 4 542 532        US-A- 4 985 922

## Description

La présente invention concerne le domaine des dispositifs médicaux implantés actifs, et plus particulièrement le recueil des signaux émis lors de séquences de communication entre le dispositif implanté et une console de contrôle extérieure.

Les dispositifs médicaux implantés actifs comprennent notamment les stimulateurs cardiaques, les défibrillateurs, les appareils neurologiques, les pompes de diffusion de substance médicale, et les implants cochléaires.

Ces appareils, une fois implantés, sont programmés de l'extérieur au moyen d'une console appelée "programmateur". La vérification des paramètres de l'implant ou la transmission d'informations enregistrées par celui-ci s'effectuent par voie électromagnétique, appelée "télémétrie" dans la technique en question. Cette console est munie d'un organe récepteur qui est placé en regard du site de l'implant. Cet organe comprend une bobine qui capte le champ magnétique en provenance de l'appareil implanté.

La présente invention propose en particulier des perfectionnements aux programmateurs qui permettent d'augmenter dans des proportions notables le rapport signal/bruit de l'organe récepteur.

L'amélioration du rapport signal/bruit est essentielle si l'on souhaite augmenter le débit de transmission des données de l'implant vers le programmateur. En effet, la vitesse maximale théorique de transmission des données est liée essentiellement à la valeur du rapport signal/bruit de la transmission.

Le débit moyen d'un appareil typique actuel est de l'ordre de $1,5.10^3$ bits par seconde dans le sens implant vers programmateur, et la quantité de données que l'on peut être amené à transmettre depuis un implant atteint couramment $8.10^3$ octets, ce qui exige donc, avec le débit d'un appareil actuel, un temps de transmission de 44 secondes.

Le perfectionnement apporté par la présente invention permet, comme on le verra, d'augmenter dans des proportions considérables ce débit, qui pourra atteindre typiquement $128.10^3$ bits par seconde dans le sens implant vers programmateur, permettant ainsi de réduire la durée du transfert d'un bloc de $8.10^3$ octets à 0,5 seconde environ seulement, au lieu des 44 secondes indiquées plus haut.

Cette augmentation considérable de débit, de près de deux ordres de grandeur, permet également d'envisager la transmission, de l'implant vers le programmateur, de volumes de données bien plus importants, tout particulièrement des enregistrements de données effectués sur une très longue période (enregistrements Holter). Ainsi, dans l'exemple d'un enregistrement d'ECG endocavitaire sur une durée de cinq heures à une cadence d'un échantillon toutes les six millisecondes avec un taux de compression de 1:10, la quantité de données à transmettre est de $2,4.10^6$ bits : avec un appareil classique n'autorisant un débit que de $1,5.10^3$ bits par seconde, la transmission de ces données exigerait 27 minutes, contre 19 secondes seulement avec une transmission à $128.10^3$ bits par seconde en utilisant un programmateur pourvu des perfectionnements de la présente invention.

Pour augmenter le rapport signal/bruit, on a déjà proposé dans le EP-A-0 661 077 (ELA Médical) de combiner les signaux captés par une pluralité de collecteurs d'onde et d'opérer une combinaison linéaire particulière des signaux délivrés par ceux-ci pour conserver essentiellement la composante utile du signal et éliminer la majeure partie des composantes de bruit en provenance de sources parasites.

Il est apparu souhaitable d'améliorer encore cette technique non seulement en réduisant le bruit par une combinaison particulière des signaux recueillis par les collecteurs d'onde, mais encore en recueillant des signaux pour lesquels la composante de bruit aura déjà été fortement réduite grâce à une géométrie particulière des collecteurs d'onde ; on peut ainsi obtenir une amélioration du rapport signal/bruit dès le recueil des signaux, avant même leur traitement par les circuits électroniques.

Pour ce faire, selon l'invention, le dispositif de réception de signaux émis par un appareil médical actif implanté, qui comporte des moyens collecteurs d'onde pour la réception d'une induction magnétique comprenant une composante utile émise par l'appareil implanté et une composante parasite d'origine externe, est caractérisé en ce que les moyens collecteurs d'onde comportent au moins une bobine réceptrice enroulée sur une première portion d'un circuit magnétique et au moins une bobine de compensation enroulée sur une seconde portion de ce circuit magnétique, ces deux portions du circuit magnétique appartenant à un élément magnétique commun et étant configurées de manière telle que, lorsque les moyens collecteurs d'onde sont disposés en vis-à-vis de l'appareil implanté, la bobine réceptrice soit essentiellement traversée une seule fois par les lignes de champ de l'induction magnétique de la composante utile et la bobine de compensation soit traversée essentiellement deux fois, en sens opposés, par cette même induction. De cette manière, on recueille aux bornes de la bobine de compensation un signal essentiellement représentatif de la composante parasite pour combinaison avec le signal recueilli aux bornes de la bobine réceptrice.

Avantageusement, l'élément magnétique est un pot de perméabilité relative supérieure à 1 comportant : un noyau central sur lequel est enroulée la bobine réceptrice ; un anneau périphérique sur lequel est enroulée la bobine de compensation ; et un corps central reliant noyau et anneau dans la région de ces derniers non tournée vers l'appareil implanté. Le matériau de l'élément magnétique est notamment un ferrite.

Dans une forme de réalisation avantageuse, il est prévu : un premier amplificateur à gain ajustable recevant un signal en provenance de la bobine réceptrice ; un second amplificateur à gain ajustable recevant un signal en provenance de la bobine de compensation ; un circuit sommateur recevant en entrée les signaux délivrés par les amplificateurs et délivrant en sortie un signal résultant ; et un circuit de commande ajustant les gains des amplificateurs pour opérer une différence entre le signal en provenance de la bobine de compensation et le signal en provenance de la bobine réceptrice de manière à essentiellement éliminer de ce dernier la composante parasite, matérialisée par le signal en provenance de la bobine de compensation.

Dans ce cas, avantageusement :

- les signaux appliqués à chacun des amplificateurs sont des signaux proportionnels aux signaux captés par les bobines respectives ou des combinaisons linéaires prédéterminées respectives des signaux en provenance des deux bobines ;
- il est prévu des moyens de sélection pour appliquer en entrée de chacun des amplificateurs, sous le contrôle des moyens de commande, soit des signaux proportionnels aux signaux captés par les bobines respectives soit des combinaisons linéaires prédéterminées respectives des signaux en provenance des deux bobines ;
- il est en outre prévu un circuit amplificateur et intégrateur à gain ajustable recevant en entrée le signal en provenance de la bobine de compensation, le circuit sommateur recevant également en entrée le signal délivré par ce circuit amplificateur et intégrateur ;
- le dispositif comprend une pluralité de bobines de compensation et des moyens de sélection de ces bobines sous le contrôle des moyens de commande.

On va maintenant décrire un exemple de mise en oeuvre de l'invention.

La figure 1 est une vue perspective, de dessous, du système de collecteurs d'onde selon l'invention.

La figure 2 est une vue en coupe du système de la figure 1 placé face à un implant et montrant la circulation des lignes de champ entre implant et collecteurs d'onde.

La figure 3 est un schéma par blocs des circuits de traitement des signaux captés par les collecteurs d'onde des figures 1 et 2.

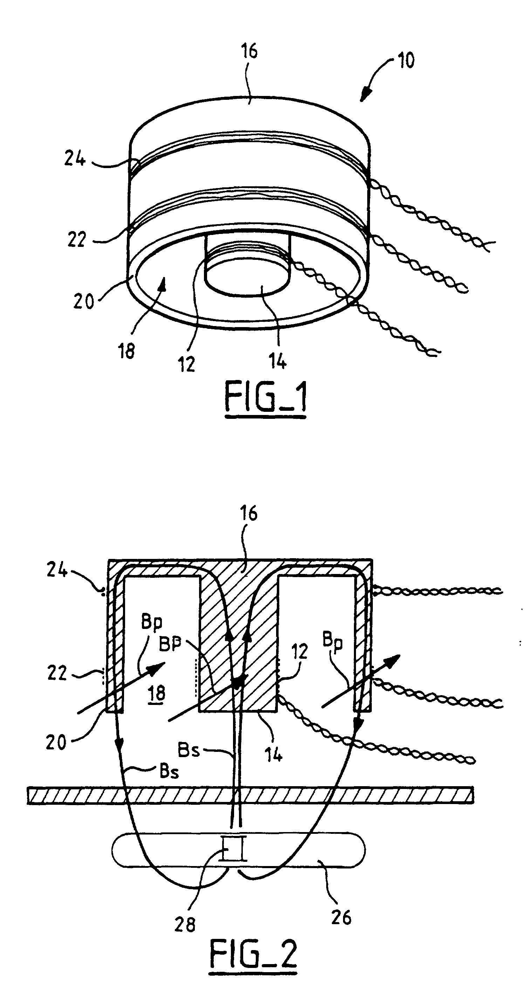

Sur les figures 1 et 2, on a représenté la structure du système de collecteurs d'onde (bobines) d'un programmateur selon l'invention.

On sait que, dans le cas de la télémétrie des informations en provenance d'un implant, les signaux utiles aussi bien que les signaux parasites sont véhiculés sous la forme de champs magnétiques, le signal total reçu par un collecteur d'onde se décomposant en un signal utile $B_S$ et un signal parasite $B_P$.

Comme cela est exposé dans le EP-A-0 661 077, il est possible d'extraire du signal total la composante de signal utile par une combinaison linéaire particulière des signaux délivrés par une pluralité de bobines distinctes recueillant chacune des flux magnétiques induits propres et produisant des tensions correspondantes appliquées à des amplificateurs respectifs à gain variable, dont les sorties sont combinées entre elles dans un étage sommateur.

La présente invention propose une géométrie particulière pour ces bobines, illustrée figures 1 et 2.

Le système de collecteurs d'onde 10 comprend au moins deux bobines, à savoir une bobine dite "réceptrice" 12, destinée essentiellement à recueillir le signal utile, et une ou plusieurs bobines dites "de compensation" destinées à recueillir essentiellement une composante de bruit.

La bobine réceptrice 12 est, de façon caractéristique de l'invention, bobinée sur le noyau central 14 d'un demi-pot (ou pot ouvert) 16 de ferrite, composant que l'on désignera simplement par commodité dans la suite par le vocable "pot".

Plus précisément, le pot 16 est un pot classique (mais dépourvu de son couvercle), de perméabilité relative supérieure à 1, comportant le noyau cylindrique central 14 entouré d'un espace annulaire 18 et d'un anneau périphérique 20. L'ensemble présente, en section radiale, une forme générale de E dont les branches sont tournées vers l'extérieur, c'est-à-dire en direction de l'implant. L'invention n'est cependant pas limitée à l'utilisation d'un ferrite sous forme de pot rond, mais elle peut être également mise en oeuvre au moyen par exemple d'un pot carré ou de toute autre forme, dès lors que la géométrie relative fonctionnelle des différents éléments, telle qu'elle sera exposée par la suite, est respectée.

L'antenne de compensation 22, quant à elle, est bobinée sur l'anneau périphérique 20 du pot de ferrite.

Le pot de ferrite porte également une bobine 24 servant à la transmission des signaux du programmateur vers l'implant. C'est par commodité que cette bobine 24 est disposée également sur le pot de ferrite 16, mais elle ne participe pas à la réception des signaux et n'est donc citée ici que pour mémoire.

Le ferrite utilisé peut avantageusement être un ferrite doux de perméabilité magnétique élevée. On sait que les matériaux de haute perméabilité magnétique ont la faculté de canaliser le flux de l'induction magnétique provoquée par des sources proches en regard de leur géométrie.

Il est par ailleurs connu que les bobines sont le siège d'une force électromotrice lorsqu'elles sont traversées par une induction magnétique variant dans le temps.

En particulier, si la bobine réceptrice 12 est traversée par une induction $B_s$ dont le flux est $\Phi_s$, et que l'on appelle SORTIE-S le signal représentatif de la force électromotrice qui y est produit par $\Phi_s$, on a :

$$\text{SORTIE-S} = - d\Phi_s/dt$$

La géométrie particulière de l'invention permet, comme on peut le voir figure 2, de placer le pot de ferrite 16 avec les antennes 12 et 22 en regard de l'implant 26 lorsqu'il est nécessaire de lire des informations transmises par celui-ci, informations qui sont par exemple véhiculées par l'induction magnétique provoquée par le passage de courants oscillants dans la bobine émettrice 28 de l'implant.

L'induction $B_S$ produite par le passage de courant dans la bobine émettrice 28 va avoir tendance, si l'ensemble est correctement positionné en face de l'implant, a entrer dans le ferrite 16 par la partie centrale 14 et à ressortir par l'anneau périphérique 20, les lignes de champ se refermant dans la bobine 28.

La bobine réceptrice 12 va donc se trouver traversée une fois et une seule par les lignes de champ de l'induction magnétique $B_S$ produite par la bobine émettrice 28, tandis que la bobine de compensation 22 va être traversée deux fois, et en sens opposé, par ces mêmes lignes de champ.

Dès lors, la bobine réceptrice centrale 12 sera essentiellement sensible aux signaux émis par l'implant, tandis que la bobine périphérique de compensation 22 ne le sera pas, ou peu.

En revanche, si des sources de courant parasites provoquent une induction parasite $B_p$ à une distance suffisante (typiquement quelques dizaines de centimètres ou plus) du pot de ferrite 16, la direction de l'induction parasite $B_p$ sera peu perturbée par le matériau du pot de ferrite 16, et cette induction traversera dans le même sens et presque identiquement les deux bobines 12 et 22, qui vont donc recueillir l'une et l'autre des composantes de bruit (composantes parasites) sensiblement identiques.

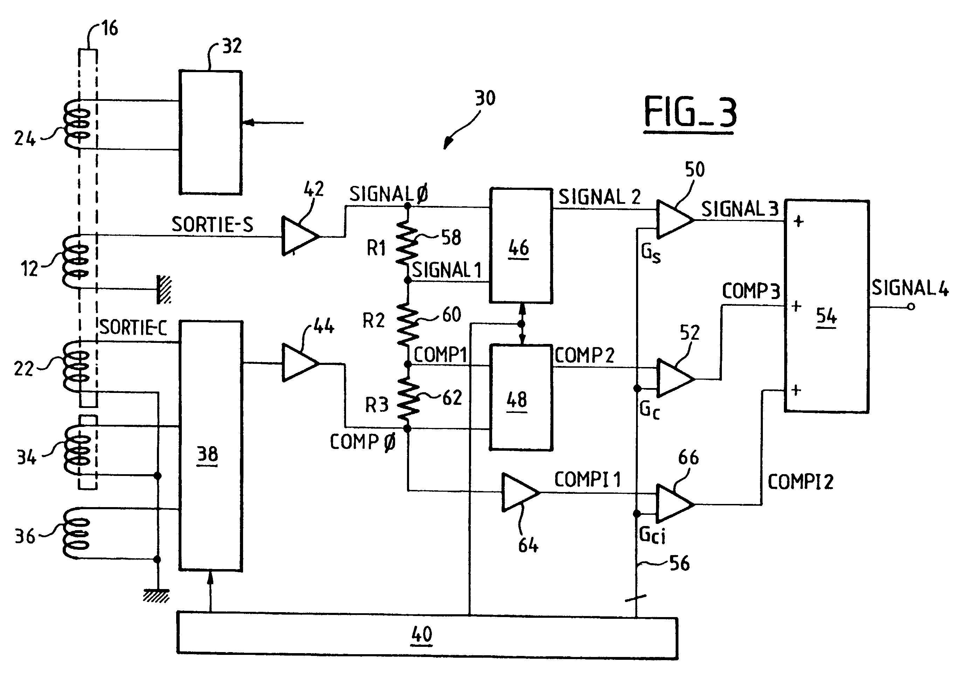

Les signaux recueillis, que l'on désignera SORTIE-S et SORTIE-C, sont alors traités, par le circuit illustré figure 3, essentiellement de la manière décrite dans le EP-A-0 661 077 précité.

Les circuits de réception sont représentés schématiquement en 30, le programmateur comportant également des circuits d'émission 32, cités ici pour mémoire seulement, qui appliquent des signaux à la bobine d'émission 24 pour permettre la communication du programmateur vers l'implant.

Bien que l'on décrira l'invention dans le cadre d'un système à deux bobines (une bobine réceptrice 12 et une bobine de compensation 22), il est possible de prévoir un nombre plus important de bobines, comme cela est enseigné dans le document antérieur précité, par exemple en installant des bobines de compensation auxiliaires telles que 34, 36 (figure 3) qui peuvent être substituées, par le biais d'un multiplexeur 38 commandé par une logique de contrôle appropriée 40, à l'antenne de compensation principale 22 en fonction des signaux reçus. Il est ainsi par exemple possible d'effectuer un essai de fonctionnement avec chacune des antennes de compensation possibles et de choisir ensuite celle qui permet de réaliser le meilleur traitement du signal, c'est-à-dire qui procure le meilleur rapport signal/bruit.

On sait, comme cela est enseigné dans le EP-A-0 661 077, qu'une combinaison linéaire de deux signaux, en l'espèce SORTIE-S et SORTIE-C, recueillis respectivement aux bornes des bobines 12 et 22 (ou 34 ou 36 si l'on utilise des bobines de compensation auxiliaires), permet d'obtenir un signal comportant pour seule composante le signal utile.

En effet, si l'on appelle $S_u(t)$ le signal utile et $S_p(t)$ le signal parasite, et que l'on écrit :

$$\text{SORTIE-S} = \alpha\, S_u(t) + \beta S_p(t)$$

$$\text{SORTIE-C} = \gamma S_u(t) + \delta S_p(t)$$

alors la somme

$$\text{SORTIE-S} - \delta/\beta\ (\text{SORTIE-C}) = (\alpha - \gamma\delta/\beta)\, S_u(t)$$

est un signal ne comportant plus de signal parasite.

Si, comme on l'a écrit plus haut, la bobine de compensation est insensible ou quasi insensible au signal, cela revient à écrire $\gamma = 0$, et la combinaison linéaire des signaux sera égale à $\alpha\, S_u(t)$, restituant donc complètement le signal utile. On optimise ainsi, dès le recueil du signal, le niveau du rapport signal/bruit.

Les signaux SORTIE-S et SORTIE-C issus respectivement de l'antenne réceptrice et de l'antenne de compensation sont tout d'abord amplifiés par les étages 42 et 44, pour donner des signaux SIGNALØ et COMPØ.

On opère ensuite sur ces signaux une combinaison linéaire pour produire un signal final exempt de parasites.

A cet effet, les signaux SIGNALØ et COMPØ sont appliqués, via des multiplexeurs 46 et 48 dont on expliquera le rôle plus loin, à des amplificateurs respectifs 50 et 52 puis à un sommateur 54.

Les amplificateurs 50 et 52 sont avantageusement des circuits incorporant des convertisseurs numérique/analogique multiplieurs ou des potentiomètres numériques ; leurs gains $G_S$ et $G_C$ sont variables et commandés par un bus 56 transportant la valeur de ces gains sous forme numérique depuis le circuit de commande 40 qui en ajuste la valeur.

Pour réaliser la combinaison linéaire souhaitée, on peut utiliser directement les signaux SIGNALØ et COMPØ. Mais, avec un même nombre de bits définissant les gains $G_S$ et $G_C$ (par exemple huit bits), on peut réaliser une compensation plus fine du signal parasite en utilisant pour la combinaison linéaire des signaux SIGNAL1 et COMP1 dérivés de SIGNALØ et COMPØ avec le pont diviseur formé des résistances 58, 60 et 62, de valeur respective $R_1$, $R_2$ et $R_3$ (avec de préférence $R_3 = R_1$). Les signaux SIGNALØ et COMPØ sont à cet effet appliqués aux deux extrémités du circuit série formé des résistances $R_1$ à $R_3$ ; le signal SIGNAL1 est prélevé entre $R_1$ et $R_2$, tandis que le signal COMP1 est prélevé entre $R_2$ et $R_3$. Il vient :

$$SIGNAL1 = 1/2 \, (SIGNALØ + COMPØ) + (SIGNALØ - COMPØ)(R_2/(2R_1+R_2))$$

$$COMP1 = 1/2 \, (SIGNALØ + COMPØ) + (SIGNALØ - COMPØ)(R_2/(2R_1+R_2))$$

Le choix des signaux SIGNAL2 et COMP2 qui seront appliqués aux amplificateurs est réalisé, respectivement entre SIGNALØ et SIGNAL1 ou COMPØ et COMP1, par les multiplexeurs 46 et 48.

Après optimisation des gains $G_S$ et $G_C$ et sélection par les multiplexeurs 38, 46 et 48 de la combinaison de signaux produisant le meilleur rapport signal/bruit, le niveau de parasites sera identique sur les signaux SIGNAL3 et COMP3 issus des amplificateurs 50 et 52, et la sommation de ces signaux par l'additionneur 54 permettra d'obtenir un signal SIGNAL4 comportant uniquement la composante de signal utile.

Toutefois, dans certaines configurations où le niveau des parasites est très intense et où la dimension de l'implant est relativement importante, il se peut que l'implant ré-émette une partie du signal parasite intégrée temporellement, du fait de courants induits prenant naissance dans la surface extérieure de l'implant, généralement formée d'une coque en matériau métallique conducteur.

Pour annuler cette composante, on sommera à SIGNAL3 et COMP3 la partie intégrée COMPI2 du signal parasite COMPØ. Pour ce faire, le signal COMPØ est appliqué en entrée d'un intégrateur 64 dont le signal de sortie COMPI1 est lui-même appliqué à un amplificateur à gain réglable 66 (du même type que les amplificateurs 50 et 52) dont le gain $G_{CI}$ est également ajusté par le circuit de commande 40.

Bien entendu, le traitement des signaux recueillis par les bobines réceptrices et de compensation peut être effectué de différentes façons, par exemple de manière entièrement analogique, ou avec une partie de circuits numériques plus ou moins importante, pilotés ou non par des moyens logiciels. En particulier, le circuit de commande 40 peut avantageusement comporter un microcalculateur apte à résoudre un système d'équations linéaires de manière à en déduire la valeur des différents gains à appliquer aux amplificateurs.

**Revendications**

**1.** Un dispositif de réception de signaux émis par un appareil médical actif implanté (26), ce dispositif comportant des moyens collecteurs d'onde (10) pour la réception d'une induction magnétique comprenant une composante utile ($B_S$) émise par l'appareil implanté et une composante parasite ($B_p$) d'origine externe,

**caractérisé en ce que** les moyens collecteurs d'onde comportent au moins une bobine réceptrice (12) enroulée sur une première portion (14) d'un circuit magnétique et au moins une bobine de compensation (22) enroulée sur une seconde portion (20) de ce circuit magnétique, ces deux portions du circuit magnétique appartenant à un élément magnétique commun (16) et étant configurées de manière telle que, lorsque les moyens collecteurs d'onde sont disposés en vis-à-vis de l'appareil implanté, la bobine réceptrice soit essentiellement traversée une seule fois par les lignes de champ de l'induction magnétique de la composante utile ($B_S$) et la bobine de compensation soit traversée essentiellement deux fois, en sens opposés, par cette même induction.

**2.** Le dispositif de la revendication 1, dans lequel l'élément magnétique est un pot (16) de perméabilité relative supérieure à 1 comportant : un noyau central (14) sur lequel est enroulée la bobine réceptrice (12) ; un anneau périphérique (20) sur lequel est enroulée la bobine de compensation (22) ; et un corps central reliant noyau et anneau dans la région de ces derniers non tournée vers l'appareil implanté (26).

3. Le dispositif de la revendication 1, dans lequel le matériau de l'élément magnétique est un ferrite.

4. Le dispositif de la revendication 1, dans lequel il est prévu : un premier amplificateur (50) à gain ($G_S$) ajustable recevant un signal (SIGNAL2) en provenance de la bobine réceptrice ; un second amplificateur (52) à gain ($G_C$) ajustable recevant un signal (COMP2) en provenance de la bobine de compensation ; un circuit sommateur (54) recevant en entrée les signaux (SIGNAL3, COMP3) délivrés par les amplificateurs et délivrant en sortie un signal résultant (SIGNAL4) ; et un circuit de commande (40) ajustant les gains ($G_S$, $G_C$) des amplificateurs pour opérer une différence entre le signal en provenance de la bobine de compensation et le signal en provenance de la bobine réceptrice de manière à essentiellement éliminer de ce dernier la composante parasite, matérialisée par le signal en provenance de la bobine de compensation.

5. Le dispositif de la revendication 4, dans lequel les signaux (SIGNAL2, COMP2) appliqués à chacun des amplificateurs sont des signaux (SIGNALØ, COMPØ) proportionnels aux signaux captés par les bobines respectives ou des combinaisons linéaires prédéterminées respectives (SIGNAL1, COMP1) des signaux en provenance des deux bobines.

6. Le dispositif de la revendication 5, dans lequel il est prévu des moyens de sélection (46, 48) pour appliquer en entrée de chacun des amplificateurs (50, 52), sous le contrôle des moyens de commande (40), soit des signaux (SIGNALØ, COMPØ) proportionnels aux signaux captés par les bobines respectives soit des combinaisons linéaires prédéterminées respectives (SIGNAL1, COMP1) des signaux en provenance des deux bobines.

7. Le dispositif de la revendication 4, dans lequel il est en outre prévu un circuit amplificateur et intégrateur (64, 66) à gain ($G_{CI}$) ajustable recevant en entrée le signal (COMPØ) en provenance de la bobine de compensation, le circuit sommateur recevant également en entrée le signal (COMPI2) délivré par ce circuit amplificateur et intégrateur.

8. Le dispositif de la revendication 4, comprenant une pluralité de bobines de compensation (22, 34, 36) et des moyens (38) de sélection de ces bobines sous le contrôle des moyens de commande (40).

**Claims**

1. A device for receiving signals transmitted by an implanted active medical appliance (26), the device comprising wave pickup means (10) for receiving magnetic induction comprising both a wanted component ($B_S$) transmitted by the implanted appliance and an interference component ($B_P$) of external origin, the device being **characterized in that** the wave pickup means comprise at least one receiver coil (12) wound on a first portion (14) of a magnetic circuit and at least one compensation coil (22) wound on a second portion (20) of the magnetic circuit, these two magnetic circuit portions belonging to a common magnetic element (16) and being configured in such a manner that, when the wave pickup means are placed in register with the implanted appliance, the receiver coil is crossed essentially once only by the field lines of the magnetic induction of the wanted component ($B_S$) while the compensation coil is crossed essentially twice by said induction, in opposite directions.

2. The device of claim 1, in which the magnetic element is a pot (16) of relative permeability greater than 1 and comprising: a central core (14) having the receiver coil (12) wound thereon; a peripheral ring (20) having the compensation coil (22) wound thereon; and a central body interconnecting the core and the ring in the region thereof that does not face towards the implanted appliance (26).

3. The device of claim 1, in which the material of the magnetic element is a ferrite.

4. The device of claim 1, in which there are provided: a first amplifier (50) of adjustable gain ($G_S$) receiving a signal (SIGNAL2) from the receiver coil; a second amplifier (52) of adjustable gain ($G_C$) receiving a signal (COMP2) from the compensation coil; a summing circuit (54) receiving as inputs the signals (SIGNAL3, COMP3) delivered by the amplifiers, and delivering a resulting output signal (SIGNAL4); and a control circuit (40) adjusting the gains ($G_S$, $G_C$) of the amplifiers so that when the difference is taken between the signal from the compensation coil and the signal from the receiver coil the interference component, as represented by the signal coming from the compensation coil, is essentially eliminated from the signal coming from the receiver coil.

5. The device of claim 4, in which the signals (SIGNAL2, COMP2) applied to each of the amplifiers are signals (SIGNALØ, COMPØ) proportional to the signals picked up by the respective coils or to predetermined respective linear combinations (SIGNAL1, COMP1) of the signals coming from the two coils.

6. The device of claim 5, in which selector means (46, 48) are provided to operate under the control of the control means (40) to apply to the inputs of each of the amplifiers (50, 52), either signals (SIGNALØ, COMPØ) proportional to the signals picked up by the respective coils, or else predetermined respective linear combinations (SIGNAL1, COMP1) of the signals coming from the two coils.

7. The device of claim 4, in which there is further provided an amplifier and integrator circuit (64, 66) of adjustable gain ($G_{CI}$) receiving as input the signal (COMPØ) from the compensation coil, with the summing circuit also receiving as input the signal (COMPI2) delivered by said amplifier and integrator circuit.

8. The device of claim 4, comprising a plurality of compensation coils (22, 34, 36) and means (38) for selecting said coils under the control of the control means (40).

**Patentansprüche**

1. Empfangsvorrichtung für Signale, die durch eine implantierte aktive medizinische Vorrichtung (26) ausgesandt werden, wobei diese Vorrichtung Wellen-Empfangsmittel (10) aufweist für den Empfang einer magnetischen Induktion, welche eine verwendbare Komponente ($B_S$) aufweist, ausgesandt durch die implantierte Vorrichtung; und eine parasitäre Komponente ($B_P$) eines externen Ursprungs, **dadurch gekennzeichnet, dass** die Wellen-Empfangsmittel zumindest eine Empfangsspule (12) aufweisen, die auf einem ersten Abschnitt (14) einer magnetischen Schaltung aufgerollt ist; und zumindest eine Kompensationsspule (22), die auf einem zweiten Abschnitt (20) dieser magnetischen Schaltung aufgerollt ist, wobei diese zwei Abschnitte der magnetischen Schaltung zu einem gemeinsamen magnetischen Element (16) gehören und auf eine Weise konfiguriert sind, dass, wenn die Wellen-Empfangsmittel gegenüberliegend zur implantierten Vorrichtung angeordnet sind, die Empfangsspule im Wesentlichen ein einziges Mal durch die Feldlinien der magnetischen Induktion der verwendbaren Komponente ($B_S$) durchquert wird und die Kompensationsspule im Wesentlichen zweimal in entgegengesetzten Richtungen durch dieselbe Induktion durchquert wird.

2. Vorrichtung nach Anspruch 1, bei welcher das magnetische Element ein Topf (16) mit einer relativen Permeabilität größer als 1 ist, aufweisend: einen zentralen Kern (14), auf dem die Empfangsspule (12) aufgerollt ist; einen peripheren Ring (20), auf dem die Kompensationsspule (22) aufgerollte ist; und einen zentralen Körper, der den Kern und den Ring in dem Bereich dieser letzteren verbindet, der nicht zur implantierten Vorrichtung (26) gedreht ist.

3. Vorrichtung nach Anspruch 1, bei welcher das Material des magnetischen Elementes ein Ferrit ist.

4. Vorrichtung nach Anspruch 1, bei welcher vorgesehen ist: ein erster Verstärker (50) mit einstellbarer Verstärkung ($G_S$), der ein Signal (SIGNAL2) mit Herkunft von der Empfangsspule empfängt; ein zweiter Verstärker (52) mit einstellbarer Verstärkung ($G_C$), der ein Signal (COMP2) mit Herkunft von der Kompensationsspule empfängt; eine Summierungsschaltung (54), welche im Eingang die Signale (SIGNAL3, COMP3) empfängt, die durch die Verstärker geliefert werden und welche als Ausgang ein resultierendes Signal (SIGNAL4) liefert; und eine Steuerschaltung (40), welche die Verstärkung ($G_S$, $G_C$) der Verstärker einstellt, um eine Differenz zwischen dem Signal mit Herkunft von der Kompensationsspule und dem Signal mit Herkunft von der Empfangsspule zu bewirken, um von diesem Letzteren die parasitäre Komponente im Wesentlichen zu eliminieren, die durch das Signal mit Herkunft von der Kompensationsspule realisiert ist.

5. Vorrichtung nach Anspruch 4, bei welcher die Signale (SIGNAL2, COMP2), die an jeden der Verstärker angelegt sind, Signale (SIGNALØ, COMPØ) sind, die proportional zu den Signalen sind, die durch die jeweiligen Spulen aufgefangen werden oder jeweilige vorherbestimmte lineare Kombinationen (SIGNAL1, COMP1) der Signale mit Herkunft von den zwei Spulen sind.

6. Vorrichtung nach Anspruch 5, bei welcher Auswahlmittel (46, 48) vorgesehen sind, um am Eingang jedes der Verstärker (50, 52) unter der Steuerung der Steuerungsmittel (40) entweder Signale (SIGNALØ, COMPØ) anzulegen, die proportional zu den durch die jeweiligen Spulen aufgefangenen Signale sind, oder jeweilige vorbestimmte lineare Kombinationen (SIGNAL1, COMP1) der Signale mit Herkunft von den zwei Spulen.

7. Vorrichtung nach Anspruch 4, bei welcher u.a. eine Verstärker- und Integrierschaltung (64, 66) vorgesehen ist mit einstellbarer Verstärkung ($G_{CI}$), welche am Eingang das Signal (COMPØ) mit Herkunft von der Kompensationsspule empfangen, wobei die Summierungsschaltung auch am Eingang das Signal (COMPI2) empfängt, das durch diese Verstärkungs- und Integrierschaltung geliefert wird.

8. Vorrichtung nach Anspruch 4, welche eine Mehrzahl von Kompensationsspulen (22, 34, 36) aufweist und Auswahlmittel (38) dieser Spulen unter der Steuerung der Steuerungsmittel (40).

FIG_1

FIG_2

FIG_3